# EUROPEAN PATENT APPLICATION

(11) **EP 2 445 026 A1**
(43) Date of publication of application: **25.04.2012**
(21) Application number: 10789415.6
(22) Date of filing: 03.06.2010
(51) Int. Cl.: H01L 51/46, C07D 333/10

(54) **ORGANIC PHOTOELECTRIC CONVERSION ELEMENT**

(30) Priority: 18.06.2009 JP 2009145140
(71) Applicant: Sumitomo Chemical Company, Limited, Tokyo 104-8260 (JP)
(72) Inventor: UETANI, Yasunori, Tsukuba-shi Ibaraki 305-0045 (JP)
(74) Representative: Vossius & Partner
(86) International application number: PCT/JP2010/059803
(87) International publication number: WO 2010/147040

(57) **Abstract**

An organic photoelectric conversion element having a high absorbance at 600 nm can be provide by a method for manufacturing an organic photoelectric conversion element having a pair of electrodes at least one of which is transparent or translucent, and an organic layer between the electrodes, the method comprising a step of applying a solution that contains a conjugated polymer compound having a thiophenediyl group as a repeating unit and a sulfur-containing heterocyclic compound on one of the electrodes to form an applied film, and a step of drying the applied film at a temperature of 70°C or less to form the organic layer.

## Description

### TECHNICAL FIELD

The present invention relates an organic photoelectric conversion element.

### BACKGROUND ART

Recently, use of organic semiconductor materials in an active layer of an organic photoelectric conversion element (organic solar battery, photosensor, and the like) has been actively examined. In particular, when a composition containing a polymer compound is used as an organic semiconductor material, an active layer can be produced by an inexpensive application method. Therefore, various methods for manufacturing an organic photoelectric conversion element containing a polymer compound have been examined. For example, in Advanced Functional Materials Vol.13 (2003) p.85 described is a method for manufacturing an organic solar battery in which a solution containing poly-3-hexylthiophene that is a conjugated polymer compound, C60PCBM that is a fullerene derivative, and 1,2-dichloroberobezene is applied on a poly(3,4-ethylenedioxythiophene) (PEDOT) layer, and dried for one hour to form an organic layer.

However, the method for manufacturing an organic photoelectric conversion element has a problem that the absorbance of an organic photoelectric conversion element at 600 nm is low unless the heating for forming the organic layer is carried out at high temperature as about 130°C under an inert atmosphere.

### SUMMARY OF THE INVENTION

The present invention provides a method capable of producing an organic photoelectric conversion element in which an organic layer is formed at a low temperature and the absorbance at 600 nm is high.

The present invention provides a method for manufacturing an organic photoelectric conversion element having a pair of electrodes at least one of which is transparent or translucent, and an organic layer disposed between the electrodes, the method comprising a step of applying a solution that contains a conjugated polymer compound having a thiophenediyl group as a repeating unit and a sulfur-containing heterocyclic compound to one of the electrodes to form an applied film; and a step of drying the applied film at a temperature of 70°C or less to form the organic layer.

### DISCLOSURE OF THE INVENTION

Hereinafter, the present invention will be described in detail.

A method for manufacturing an organic photoelectric conversion element of the present invention comprises a step of applying a solution that contains a conjugated polymer compound having a thiophenediyl group as a repeating unit and a sulfur-containing heterocyclic compound to one electrode to form an applied film; and a step of drying the applied film at a temperature of 70°C or less to form an organic layer.

The use of the method for manufacturing an organic photoelectric conversion element of the present invention causes crystallization of the conjugated polymer compound having a thiophenediyl group in the organic layer, and the absorption wavelength of the conjugated polymer compound having a thiophenediyl group is shifted toward to a longer wavelength. As a result, the absorbance of the organic photoelectric conversion element at 600 nm becomes higher. The higher absorbance at 600 nm is a factor for enhancing the photoelectric conversion efficiency of the organic photoelectric conversion element. Since the method for manufacturing an organic photoelectric conversion element of the present invention promotes crystallization of the conjugated polymer compound having a thiophenediyl group without adopting a temperature condition of more than 70°C, energy necessary for manufacturing can be reduced.

It is preferable that the sulfur-containing heterocyclic compound used in the present invention has a condensed polycyclic structure or a bithiophene structure.

The sulfur-containing heterocyclic compound having a bithiophene structure includes a compound represented by formula (1), a compound represented by formula (2), and the like. 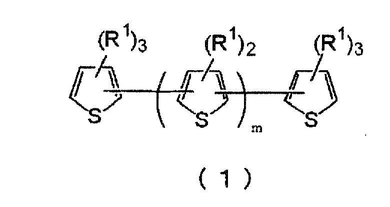 [in the formula, a plurality of R¹s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group. A hydrogen atom(s) contained in these groups may be substituted with a fluorine atom(s). m denotes an integer from 0 to 10.] 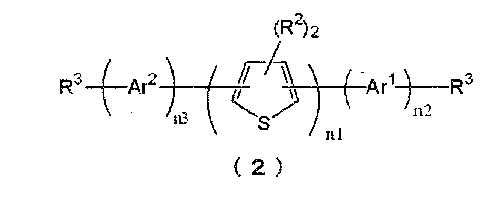 [in the formula, a plurality of R²s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group. A hydrogen atom(s) contained in these groups may be substituted with a fluorine atom(s). A plurality of R³s may be the same or different, and denote a hydrogen atom, a halogen atom, an alkyl group, an alkoxy group or an alkylthio group. A hydrogen atom(s) contained in these groups may be substituted with a fluorine atom(s). Ar¹ and Ar² independently denote an arylene group or a divalent nitrogen-containing aromatic heterocyclic group. n1 denotes an integer from 2 to 10, n2 denotes an integer from 1 to 3, and n3 denotes an integer from 1 to 3. When a plurality of Ar¹s are present, they may be the same or different, and when a plurality of Ar²s are present, they may be the same or different.]

In formula (1), the halogen atom represented by R¹ may be a fluorine atom, a chlorine atom, a bromine atom, and an iodine atom.

In formula (1), the alkyl group represented by R¹ may be linear or branched, or may be a cycloalkyl group, and usually has 1 to 20 carbon atoms. Specific examples of the alkyl group include a methyl group, an ethyl group, a n-propyl group, an isopropyl group, a n-butyl group, an isobutyl group, a t-butyl group, a s-butyl group, a 3-methyl butyl group, a n-pentyl group, a n-hexyl group, a 2-ethylhexyl group, a n-heptyl group, a n-octyl group, a n-nonyl group, a n-decyl group, a 3,7-dimethyloctyl group, and a n-lauryl group. A hydrogen atom(s) in the alkyl group may be substituted with a fluorine atom(s). Examples of the alkyl group substituted with a fluorine atom(s) include a trifluoromethyl group, a pentafluoroethyl group, a perfluorobutyl group, a perfluorohexyl group, and a perfluorooctyl group.

In formula (1), the alkoxy group represented by R¹ may be linear or branched, or may be a cycloalkyloxy group, and usually has 1 to 20 carbon atoms. Specific examples of the alkoxy group include a methoxy group, an ethoxy group, a n-propyloxy group, an isopropyloxy group, a n-butoxy group, an i-butoxy group, a s-butoxy group, a t-butoxy group, a n-pentyloxy group, a n-hexyloxy group, a cyclohexyloxy group, a n-heptyloxy group, a n-octyloxy group, a 2-ethylhexyloxy group, a n-nonyloxy group, a n-decyloxy group, a 3,7-dimethyloctyloxy group, and a n-lauryloxy group. A hydrogen atom(s) in the alkoxy group may be substituted with a fluorine atom(s). Examples of the alkoxy group substituted with a fluorine atom (s) include a trifluoromethoxy group, a pentafluoroethoxy group, a perfluorobutoxy group, a perfluorohexyl group, and a perfluorooctyl group.

In formula (1), the alkylthio group represented by R¹ may be linear or branched, or may be a cycloalkylthio group, and usually has 1 to 20 carbon atoms. Specific examples of the alkylthio group include a methylthio group, an ethylthio group, a n-propylthio group, an isopropylthio group, a n-butylthio group, an isobutylthio group, a s-butylthio group, a t-butylthio group, a n-pentylthio group, a n-hexylthio group, a cyclohexylthio group, a n-heptylthio group, a n-octylthio group, a 2--ethylhexylthio group, a n-nonylthio group, a n-decylthio group, a 3,7-dimethyloctylthio group, and a n-laurylthio group. A hydrogen atom(s) in the alkylthio group may be substituted with a fluorine atom(s). Examples of the alkylthio group substituted with a fluorine atom(s) include a trifluoromethylthio group.

In formula (1), the aryl group represented by R¹ is an atomic group in which one hydrogen atom is removed from an aromatic hydrocarbon, and includes one having a benzene ring, one having a condensed ring, and one to which an independent benzene ring or two or more condensed rings are bonded directly or bonded via a group such as vinylene. The aryl group usually has 6 to 60 carbon atoms, and preferably has 6 to 48 carbon atoms. The aryl group may have a substituent(s). Examples of the substituent include a linear or branched alkyl group having 1 to 20 carbon atoms or a cycloalkyl group having 1 to 20 carbon atoms, an alkoxy group including a linear or branched alkyl group having 1 to 20 carbon atoms or a cycloalkyl group having 1 to 20 carbon atoms in its structure, and a group represented by formula (5): 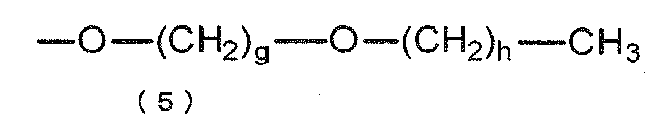 (in formula (5), g denotes an integer from 1 to 6, and h denotes an integer from 0 to 5). Specific examples of the aryl group that may have a substituent(s) include a phenyl group, a C₁-C₁₂ alkoxyphenyl group (C₁-C₁₂ denotes that the number of carbon atoms is from 1 to 12. The same is true hereinafter.), a C₁-C₁₂ alkylphenyl group, a 1-naphthyl group, a 2-naphthyl group, a 1-anthracenyl group, a 2-anthracenyl group, a 9-anthracenyl group, and a pentafluorophenyl group. A C₁-C₁₂ alkoxyphenyl group and a C₁-C₁₂ alkylphenyl group are preferable. Specific examples of the C₁-C₁₂ alkoxyphenyl group include a methoxyphenyl group, an ethoxyphenyl group, a n-propyloxyphenyl group, an isopropyloxyphenyl group, a n-butoxyphenyl group, an isobutoxyphenyl group, a s-butoxyphenyl group, a t-butoxyphenyl group, a n-pentyloxyphenyl group, a n-hexyloxyphenyl group, a cyclohexyloxyphenyl group, a n-heptyloxyphenyl group, a n-octyloxyphenyl group, a 2-ethylhexyloxyphenyl group, a n-nonyloxyphenyl group, a n-decyloxyphenyl group, a 3,7-dimethyloctyloxyphenyl group, and a n-lauryloxyphenyl group. Specific examples of the C₁-C₁₂ alkylphenyl group include a methylphenyl group, an ethylphenyl group, a dimethylphenyl group, a n-propylphenyl group, a mesityl group, a methylethylphenyl group, an isopropylphenyl group, a n-butylphenyl group, an isobutylphenyl group, a s-butylphenyl group, a t-butylphenyl group, a n-pentylphenyl group, an isoamylphenyl group, a hexylphenyl group, a n-heptylphenyl group, a n-octylphenyl group, a n-nonylphenyl group, a n-decylphenyl group, and a n-dodecylphenyl group. A hydrogen atom(s) in the aryl group may be substituted with a fluorine atom(s).

In formula (1), the aryloxy group represented by R¹ usually has 6 to 60 carbon atoms, and preferably has 6 to 48 carbon atoms. Specific examples of the aryloxy group include a phenoxy group, a C₁-C₁₂ alkoxyphenoxy group, a C₁-C₁₂ alkylphenoxy group, a 1-naphthyloxy group, a 2-naphthyloxy group, and a pentafluorophenyloxy group. A C₁-C₁₂ alkoxyphenoxy group and a C₁-C₁₂ alkylphenoxy group are preferable. Specific examples of the C₁-C₁₂ alkoxyphenoxy group include a methoxyphenoxy group, an ethoxyphenoxy group, a n-propyloxyphenoxy group, an isopropyloxyphenoxy group, a n-butoxyphenoxy group, an isobtoxyphenoxy group, a s-butoxyphenoxy group, a t-butoxyphenoxy group, a n-pentyloxyphenoxy group, a n-hexyloxyphenoxy group, a cyclohexyloxyphenoxy group, a n-heptyloxyphenoxy group, a n-octyloxyphenoxy group, a 2-ethylhexyloxyphenoxy group, a n-nonyloxyphenoxy group, a n-decyloxyphenoxy group, a 3,7-dimethyloctyloxyphenoxy group, and a n-lauryloxyphenoxy group. Examples of the C₁-C₁₂ alkylphenoxy group include a methylphenoxy group, an ethylphenoxy group, a dimethylphenoxy group, a n-propylphenoxy group, a 1,3,5-trimethylphenoxy group, a methylethylphenoxy group, an isopropylphenoxy group, a n-butylphenoxy group, an isobutylphenoxy group, a s-butylphenoxy group, a t-butylphenoxy group, a n-pentylphenoxy group, an isoamylphenoxy group, a n-hexylphenoxy group, a n-heptylphenoxy group, a n-octylphenoxy group, a n-nonylphenoxy group, a n-decylphenoxy group, and a n-dodecylphenoxy group.

In formula (1), the arylthio group represented by R¹ may have a substituent (s) on an aromatic ring and usually has 6 to 60 carbon atoms. Specific examples of the arylthio group include a phenylthio group, a C₁-C₁₂ alkoxyphenylthio group, a C₁-C₁₂ alkylphenylthio group, a 1-naphthylthio group, a 2-naphthylthio group, a pentafluorophenylthio group, a pyridylthio group, a pyridazinylthio group, a pyrimidylthio group, a pyrazylthio group, and a triazylthio group.

In formula (1), the arylalkyl group represented by R¹ may have a substituent(s) and usually has 7 to 60 carbon atoms. Specific examples of the arylalkyl group include a phenyl-C₁-C₁₂ alkyl group, a C₁-C₁₂ alkoxyphenyl-C₁-C₁₂ alkyl group, a C₁-C₁₂ alkylphenyl-C₁-C₁₂ alkyl group, a 1-naphthvl-C₁-C₁₂ alkyl group, and a 2-naphthyl-C₁-C₁₂ alkyl group.

In formula (1), the arylalkoxy group represented by R¹ may have a substituent (s) and usually has 7 to 60 carbon atoms. Specific examples of the arylalkoxy group include a phenyl-C₁-C₁₂ alkoxy group, a C₁-C₁₂ alkoxyphenyl-C₁-C₁₂ alkoxy group, a C₁-C₁₂ alkylphenyl-C₁-C₁₂ alkoxy group, a 1-naphthyl-C₁-C₁₂ alkoxy group, and a 2-naphthyl-C₁-C₁₂ alkoxy group.

In formula (1), the arylalkylthio group represented by R¹ may have a substituent (s) and usually has 7 to 60 carbon atoms. Specific examples of the arylalkylthio group include a phenyl-C₁-C₁₂ alkylthio group, a C₁-C₁₂ al koxyphenyl-C₁-C₁₂ alkylthio group, a C₁-C₁₂ alkylphenyl-C₁-C₁₂ alkylthio group, a 1-naphthyl-C₁-C₁₂ alkylthio group, and a 2-naphthyl-C₁-C₁₂ alkylthio group.

From the viewpoint of a charge transport property, R¹ is preferably a hydrogen atom, an alkyl group, and a halogen atom.

Specific examples of the compound represented by formula (1) are as follows.

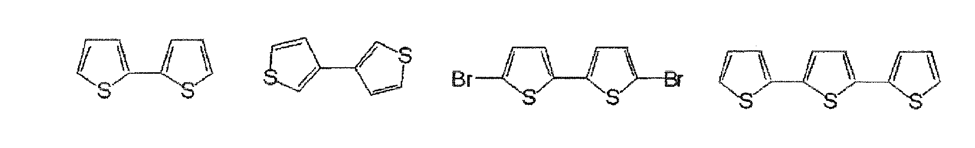

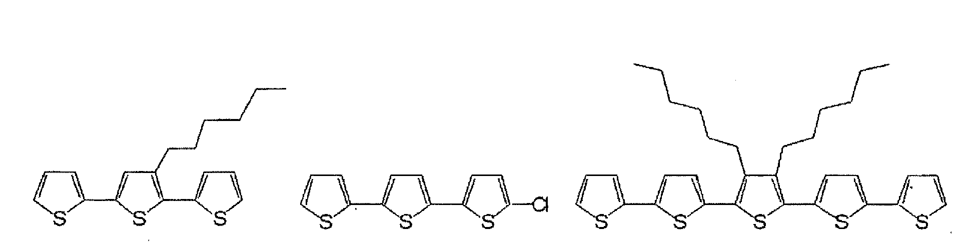

In formula (2), the alkyl group, the alkoxy group, the alkylthio group, the aryl group, the aryloxy group, the arylthio group, the arylalkyl group, the arylalkoxy group, and the arylalkylthio group represented by R² are exemplified by the same groups as those in the case of R¹ mentioned above. Furthermore, the halogen atom represented by R² is exemplified by the same atoms as those in the case of R¹ mentioned above.

From the viewpoint of the charge transport property, R² is preferably a hydrogen atom, an alkyl group, and a halogen atom.

In formula (2), the alkyl group, the alkoxy group, and the alkylthio group represented by R³ are exemplified by the same groups as those in the case of R¹ mentioned above. Furthermore, the halogen atom represented by R³ is exemplified by the same atoms as those in the case of R¹ mentioned above.

From the viewpoint of the charge transport property, R³ is preferably an alkyl group and a halogen atom.

In formula (2), Ar¹ and Ar² independently denote an arylene group or a divalent nitrogen-containing aromatic heterocyclic group.

Herein, the arylene group is an atomic group in which two hydrogen atoms are removed from an aromatic hydrocarbon, and examples thereof include one having a benzene ring, one having a condensed ring, and one to which an independent benzene ring or two or more condensed rings are bonded directly or bonded via a group such as vinylene. The arylene group may have a substituent(s). Examples of the substituent include a linear or branched alkyl group having 1 to 20 carbon atoms or a cycloalkyl group having 1 to 20 carbon atoms, and an alkoxy group including a linear or branched alkyl group having 1 to 20 carbon atoms or a cycloalkyl group having 1 to 20 carbon atoms in its structure. A part in which the substituent is removed in the arylene group usually has about 6 to 60 carbon atoms, and preferably has 6 to 20 carbon atoms. Furthermore, the total number of carbon atoms of the arylene group including the substituent is usually about 6 to 100.

Examples of the arylene group include phenylene groups (formulae 1 to 3), naphthalenediyl groups (formulae 4 to 13), anthracenediyl groups (formulae 14 to 19), biphenyldiyl groups (formulae 20 to 25), terphenyldiyl groups (formulae 26 to 28), condensed ring compound groups (formulae 29 to 35), a fluorenediyl group (formula 36), benzofluorenediyl groups (formulae 37 to 39), and a dibenzofluorenediyl group (formula 40). 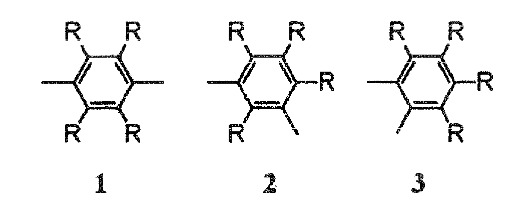 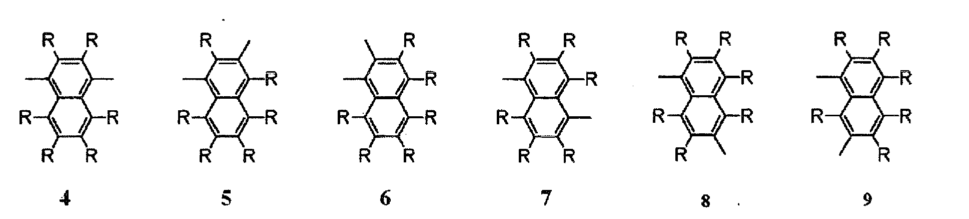 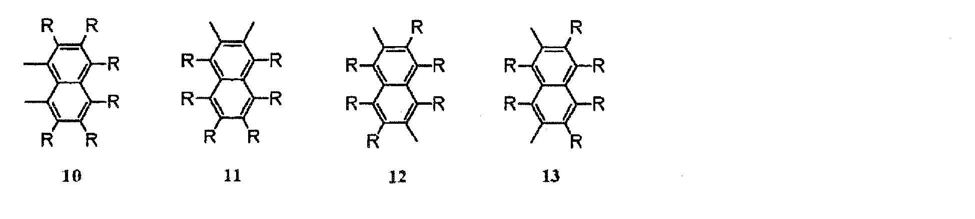 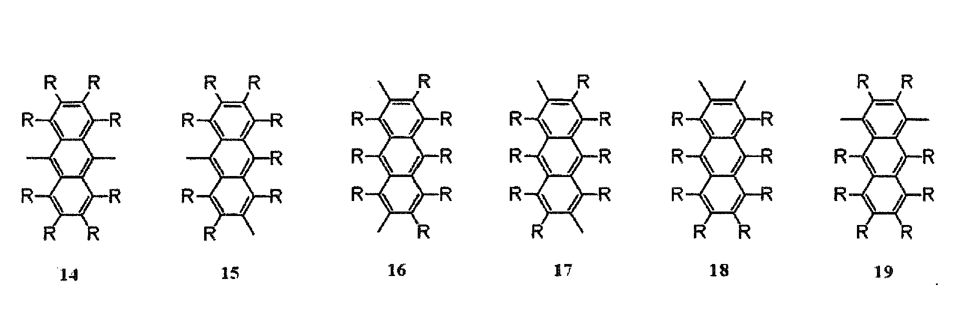 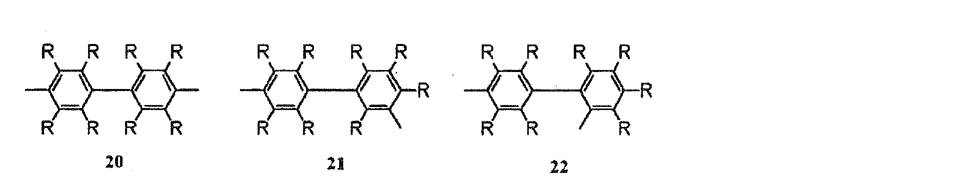 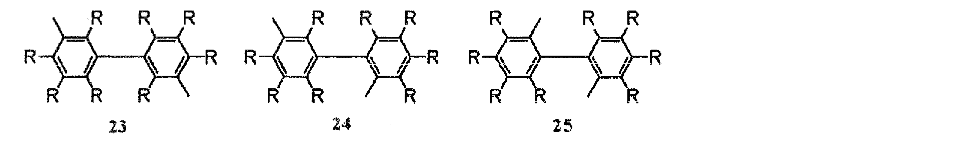 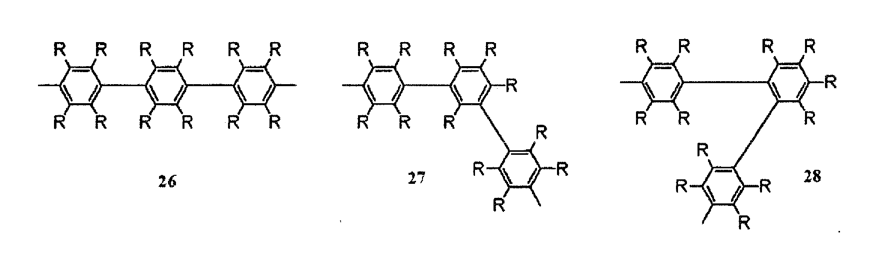 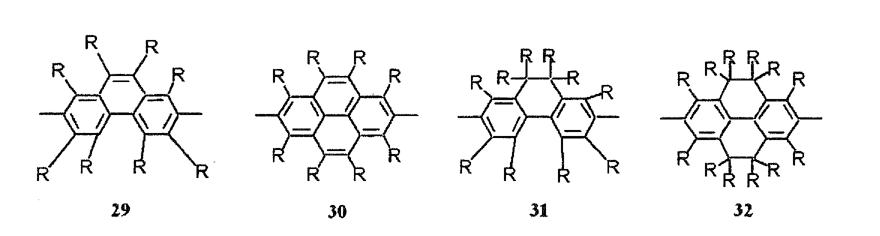 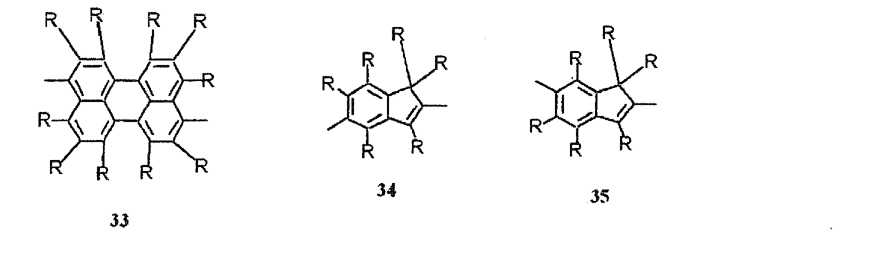 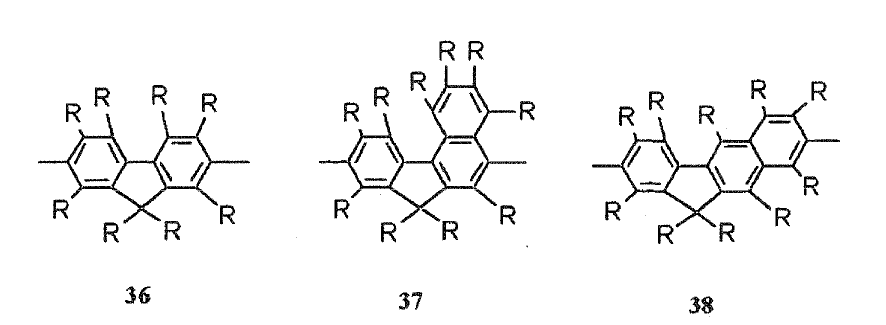 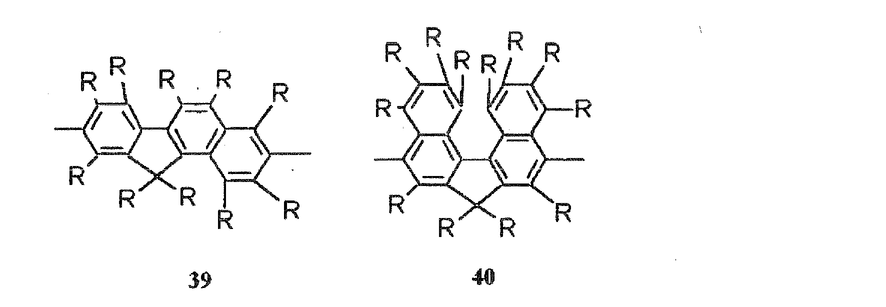 [in the formulae, R denotes a hydrogen atom, a halogen atom, an alkyl group, an alkoxy group, an alkylthio group, an aryl group, an aryloxy group, an arylthio group, an arylalkyl group, an arylalkoxy group, or an arylalkylthio group. A plurality of Rs may be the same or different.)

In formulae 1 to 40, the alkyl group, the alkoxy group, the alkylthio group, the aryl group, the aryloxy group, the arylthio group, an arylalkyl group, the arylalkoxy group, and the arylalkylthio group represented by R are exemplified by the same groups as those in the case of R¹ mentioned above. Furthermore, the halogen atom represented by R is exemplified by the same atoms as those in the case of R¹ mentioned above.

Herein, the divalent nitrogen-containing aromatic heterocyclic group means an atomic group in which two hydrogen atoms are removed from an aromatic heterocyclic compound having a nitrogen atom in the ring. The heterocyclic group may have a substituent (s). Furthermore, the aromatic heterocyclic compound having a nitrogen atom in the ring may further include an oxygen atom, a sulfur atom, a selenium atom, and the like in the ring. A part in which a substituent is removed in the heterocyclic group usually has about 3 to 60 carbon atoms. Furthermore, the total number of carbon atoms of the heterocyclic group including the substituent is usually 3 to 100.

Specific examples of the divalent nitrogen-containing aromatic heterocyclic group include pyridinediyl groups (formulae 101 to 106), diazaphenylene groups (formulae 107 to 110), quinolinediyl groups (formulae 111 to 125), quinoxalinediyl groups (formulae 126 to 130), acridinediyl groups (formulae 131 to 134), bipyridyldiyl groups (formulae 135 to 137), phenanthrolinediyl groups (formulae 138 to 140), a five-membered nitrogen-containing heterocyclic group (formula 141), and five-membered nitrogen-containing condensed heterocyclic groups (the following formulae 142 to 149). 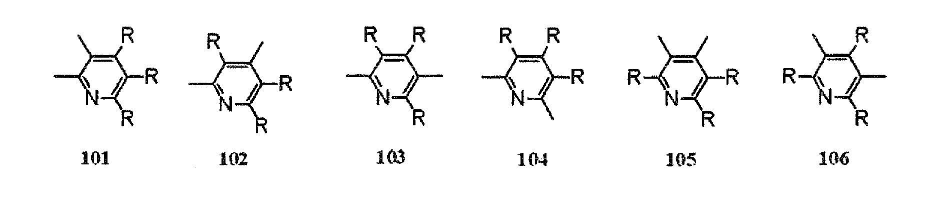 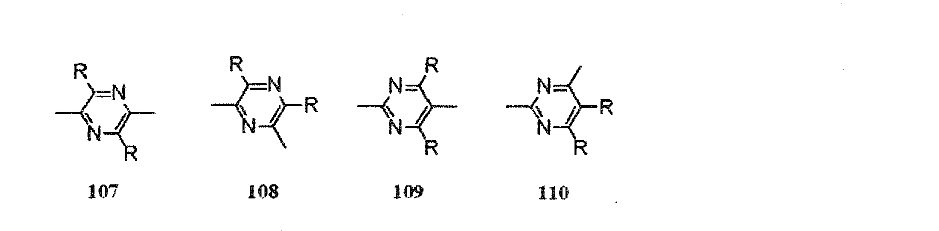 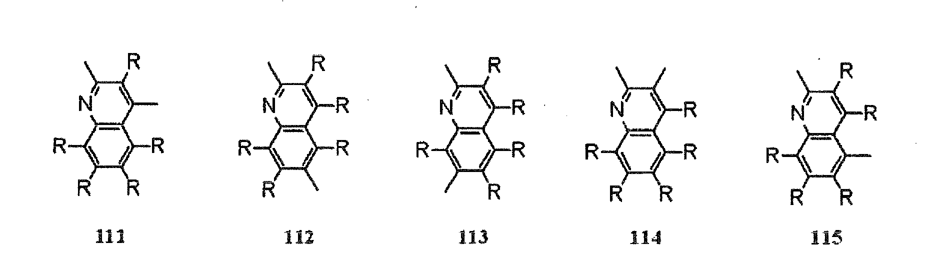 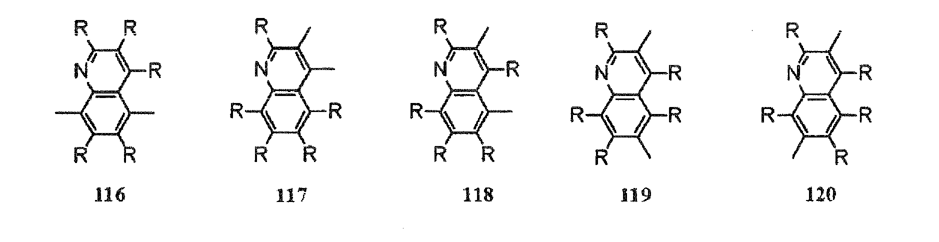 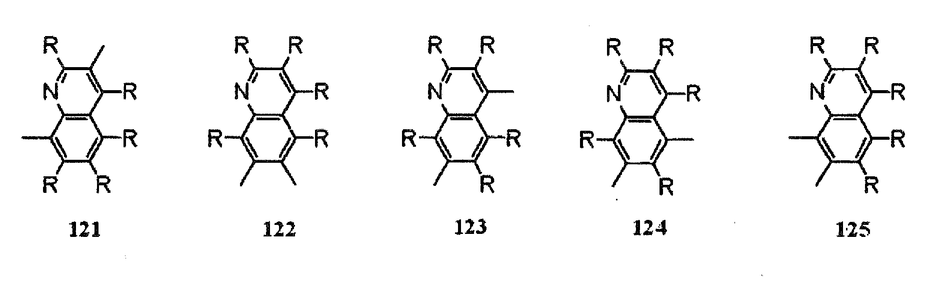 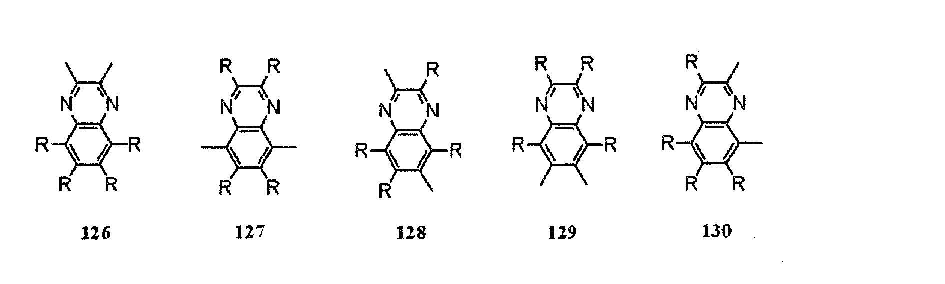 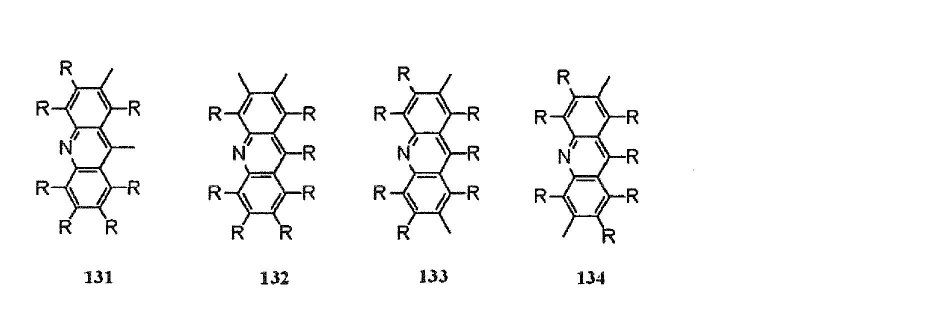 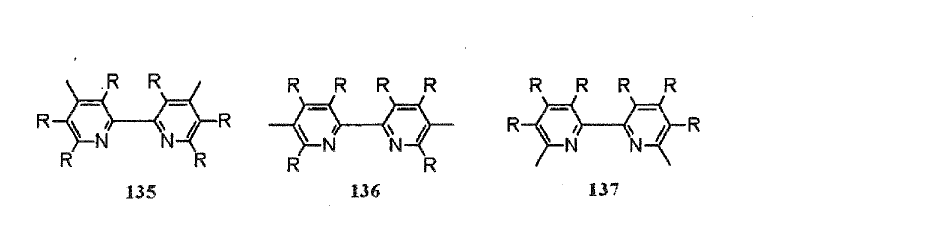 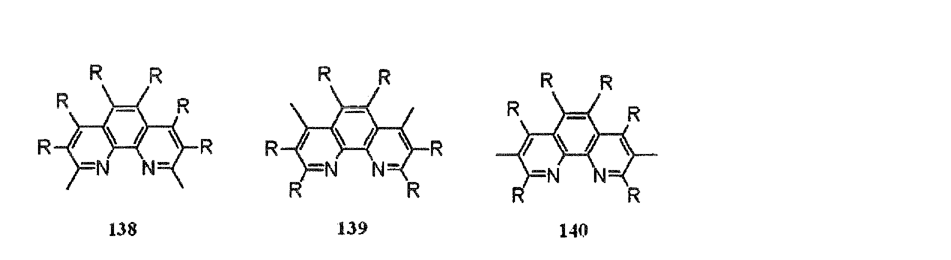 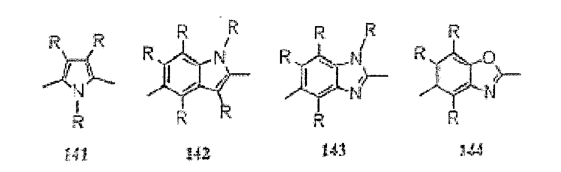 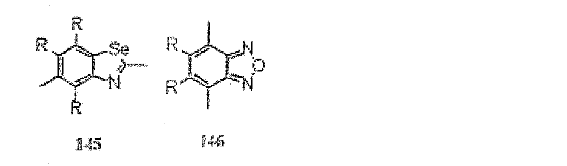 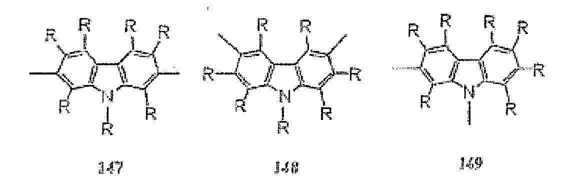 [in the formulae, R is defined as mentioned above. A plurality of Rs may be the same or may be different.)

Examples of the compound represented by formula (2) include the following compounds.

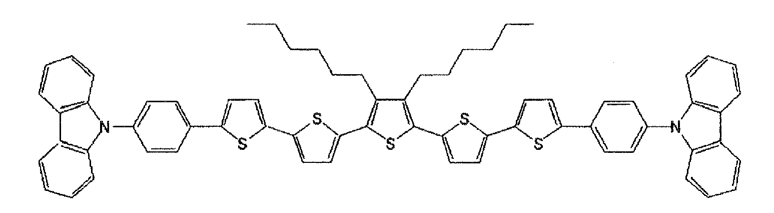

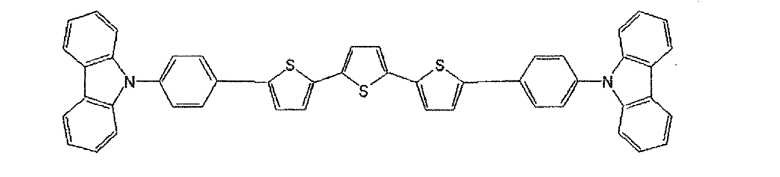

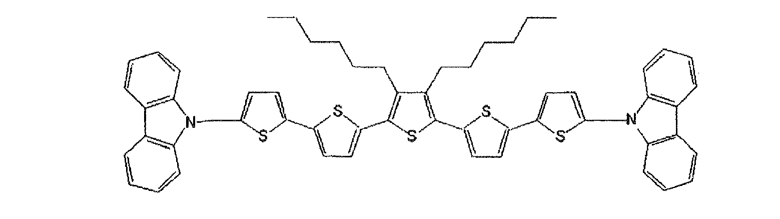

The sulfur-containing heterocyclic compound used in the present invention may have a condensed polycyclic means a structure in which two or more aromatic rings are condensed (condensed ring). It is preferable that the condensed polycyclic structure includes a sulfur atom, and it is more preferable that the condensed polycyclic structure includes 1 to 3 sulfur atoms. Examples of the aromatic ring include a thiophene ring, a benzene ring, a furan ring, a pyrrole ring, a pyridine ring, a thiadiazole ring, and a thiazole ring. The number of condensed aromatic rings is preferably 2 to 20, more preferably 2 to 20, and further preferably 2 to 5. The condensed polycyclic structure can be a structure in which two thiophene rings are condensed, a structure in which a thiophene ring and a benzene ring are condensed, a structure in which a thiadiazole ring and a benzene ring are condensed, a structure in which a thiazole ring and a benzene ring are condensed, a structure in which a thiophene ring and two benzene rings are condensed, a structure in which two thiophene rings and a benzene ring are condensed, a structure in which two thiophene rings and two benzene rings are condensed, and the like.

Examples of the sulfur-containing heterocyclic compound having a condensed polycyclic structure include a compound represented by formula (3) and a compound represented by formula (4). 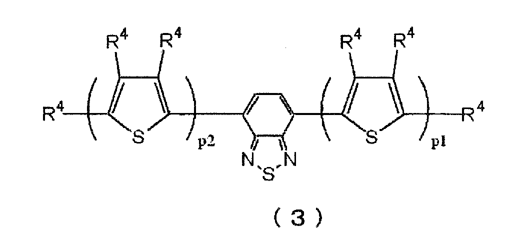 [in the formula, a plurality of R⁴s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group. A hydrogen atom(s) contained in these groups may be substituted with a fluorine atom(s). p1 denotes an integer from 0 to 5, and p2 denotes an integer from 0 to 5.] 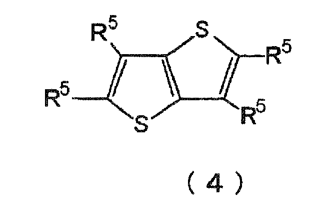 [in the formula, a plurality of R⁵s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group. A hydrogen atom(s) contained in these groups may be substituted with a fluorine atom(s).]

In formula (3), the alkyl group, the alkoxy group, the alkylthio group, the aryl group, the aryloxy group, the arylthio group, the arylalkyl group, the arylalkoxy group, and the arylalkylthio group represented by R⁴ are exemplified by the same as those in the case of R¹ mentioned above. Furthermore, the halogen atom represented by R⁴ is exemplified by the same atoms as those in the case of R¹ mentioned above.

From the viewpoint of the charge transport property, R⁴ is preferably a hydrogen atom, an alkyl group, and a halogen atom.

Specific examples of the compound represented by formula (3) are as follows.

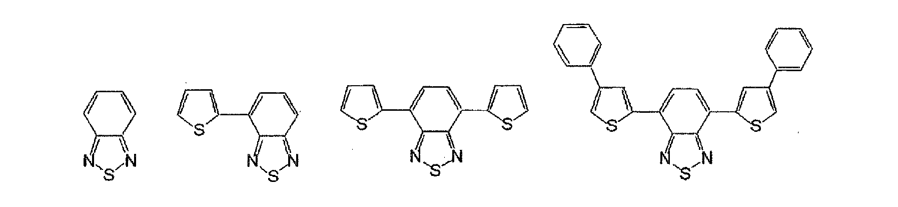

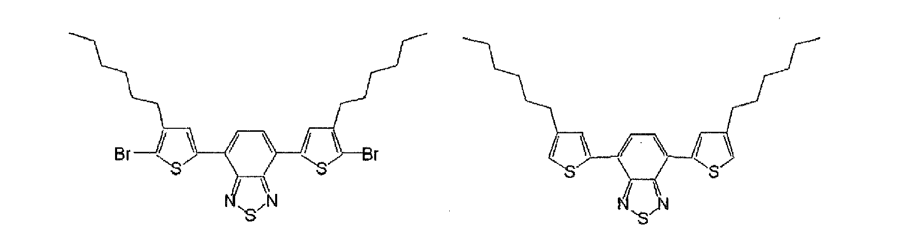

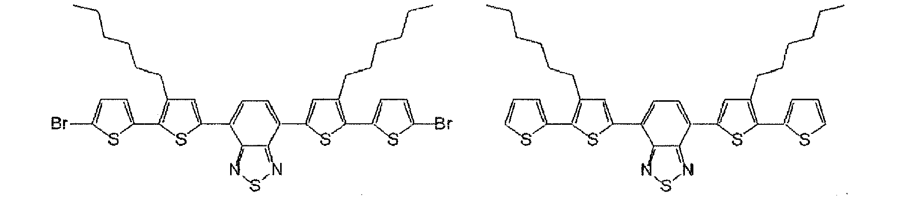

In formula (4), the alkyl group, the alkoxy group, the alkylthio group, the aryl group, the aryloxy group, the arylthio group, the arylalkyl group, the arylalkoxy group, and the arylalkylthio group represented by R⁵ are exemplified by the same groups as those in the case of R¹ mentioned above. Furthermore, the halogen atom represented by R⁵ is exemplified by the same atoms as those in the case of R¹ mentioned above.

From the viewpoint of the charge transport property, R⁵ is preferably a hydrogen atom, an alkyl group, and a halogen atom.

Specific examples of the compound represented by formula (4) are as follows.

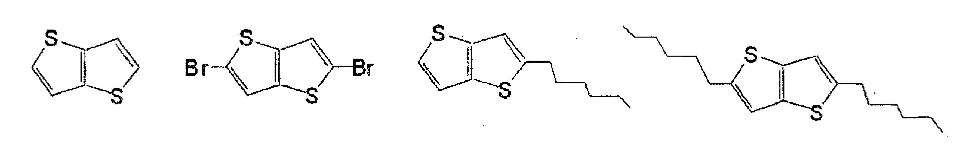

In the method for manufacturing an organic photoelectric conversion element of the present invention, the sulfur-containing heterocyclic compound contained in the organic layer of the organic photoelectric conversion element may be sole or two or more.

The conjugated polymer compound used in the method for manufacturing an organic photoelectric conversion element of the present invention has a thiophenediyl group as a repeating unit. Herein, the conjugated polymer compound means: (1) a polymer consisting essentially of a structure in which a double bond and a single bond are alternately arranged, (2) a polymer consisting essentially of a structure in which a double bond and a single bond are arranged via a nitrogen atom, (3) a polymer consisting essentially of a structure in which a double bond and a single bond are alternately arranged, and a structure in which a double bond and a single bond are arranged via a nitrogen atom; and the like. Examples of the conjugated polymer compound include a polymer having one or more groups selected from the group consisting of a unsubstituted or substituted fluorenediyl group, an unsubstituted or substituted benzofluorenediyl group, an unsubstituted or substituted dibenzofurandiyl group, an unsubstituted or substituted dibenzothiophenediyl group, an unsubstituted or substituted carbazolediyl group, an unsubstituted or substituted furandiyl group, an unsubstituted or substituted pyrrolediyl group, an unsubstituted or substituted benzothiadiazolediyl group, an unsubstituted or substituted phenylenevinylene group, an unsubstituted or substituted thienylenevinylene group, and an unsubstituted or substituted triphenylaminediyl group, as well as a thiophenediyl group, as repeating units, in which the repeating units are bonded directly or bonded via a connecting group.

In the conjugated polymer compound having a thiophenediyl group, when the repeating units are bonded via the connecting group, examples of the connecting group include phenylene, biphenylene, naphthalenediyl, and anthracenediyl.

From the viewpoint of the charge transport property, the conjugated polymer compound having a thiophenediyl group, which is used in the present invention, preferably has a repeating unit represented by formula (6), or preferably has the repeating unit represented by formula (6) and a repeating unit represented by formula (7). 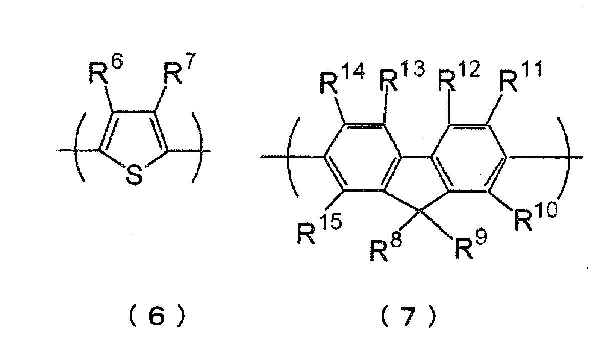 [in the formulae, R⁶, R⁷, R⁸, R⁹, R¹⁰, R¹¹, R¹², R¹³, R¹⁴, and R¹⁵ independently denote a hydrogen atom, an alkyl group, an alkoxy group, an alkylthio group, an aryl group, an aryloxy group, an arylthio group, an arylalkyl group, an arylalkoxy group, or an arylalkylthio group.]

The alkyl group, the alkoxy group, the alkylthio group, the aryl group, the aryloxy group, the arylthio group, the arylalkyl group, the arylalkoxy group, and the arylalkylthio group represented by R⁶ to R¹⁵ are exemplified by the same groups as those in the case of R¹ mentioned above.

From the viewpoints of the film forming ability and the solubility to a solvent, the conjugated polymer compound having a thiophenediyl group has a polystyrene-equivalent weight average molecular weight of preferably 5 × 10² to 1 × 10⁷, and more preferably 1 × 10³ to 1 × 10⁶.

The conjugated polymer compound having a thiophenediyl group, which is contained in the organic layer of the organic photoelectric conversion element of the present invention, may be sole or two or more.

The conjugated polymer compound having a thiophenediyl group can be produced by preparing a monomer having a functional group suitable for a polymerization reaction to be used, dissolving the monomer in an organic solvent as needed, and polymerizing it by a known polymerization method such as aryl coupling using, for example, alkali, a suitable catalyst, and a ligand.

Among the conjugated polymer compounds having a thiophenediyl group, regioregular poly-3-substituted thiophene is preferable.

The regioregular poly-3-substituted thiophene that can be used for the present invention has a repeating unit represented by formula (8). 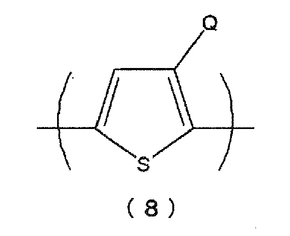 [in the formula, Q denotes an alkyl group, an alkoxy group, an alkylthio group, an aryl group, an aryloxy group, an arylthio group, an arylalkyl group, an arylalkoxy group, or an arylalkylthio group.]

The alkyl group, the alkoxy group, the alkylthio group, the aryl group, the aryloxy group, the arylthio group, the arylalkyl group, the arylalkoxy group, and the arylalkylthio group represented by Q are exemplified by the same groups as those in the case of R¹ mentioned above.

In the present invention, a solution containing a conjugated polymer compound having a thiophenediyl group and a sulfur-containing heterocyclic compound is applied on one electrode so as to form an applied film. A solvent used in the solution is not particularly limited as long as it dissolves the conjugated polymer compound having a thiophenediyl group and the sulfur-containing heterocyclic compound. Specific examples thereof include unsaturated hydrocarbon solvents such as toluene, xylene, mesitylene, tetralin, decalin, bicyclohexyl, n-butylbenzene, sec-butylbenzene, and tert-butylbenzene; halogenated saturated hydrocarbon solvents such as carbon tetrachloride, chloroform, dichloromethane, dichloroethane, chlorobutane, bromobutane, chloropentane, bromopentane, chlorohexane, bromohexane, chlorocyclohexane, and bromocyclohexane; halogenated unsaturated hydrocarbon solvents such as chlorobenzene, dichlorobenzene, and trichlorobenzene; and ether solvents such as tetrahydrofuran and tetrahydropyran. The total amount of the sulfur-containing heterocyclic compound and the conjugated polymer compound having a thiophenediyl group in the solution is usually 0.1% by weight or more.

The method for manufacturing an organic photoelectric conversion element of the present invention comprises a step of applying a solution containing a conjugated polymer compound having a thiophenediyl group as a repeating unit and a sulfur-containing heterocyclic compound on one electrode to form an applied film, and a step of drying the applied film at 70°C or less to form an organic layer. The solution used in the present invention can be prepared by dissolving the sulfur-containing heterocyclic compound and the conjugated polymer compound having a thiophenediyl group in a solvent. When an electron-accepting compound is also contained in the organic layer, an electron-accepting compound may be further dissolved in the solution. Furthermore, when an electron-donating compound is contained in the organic layer, an electron-donating compound may be further dissolved in the solution.

The weight of the sulfur-containing heterocyclic compound in the solution is preferably 1 to 10000 parts by weight, and more preferably 1 to 1000 parts by weight with respect to 100 parts by weight of the conjugated polymer compound having a thiophenediyl group. When an electron-accepting compound is contained in the solution, the amount of the electron-accepting compound is preferably 1 to 10000 parts by weight, and more preferably 10 to 2000 parts by weight with respect to 100 parts by weight of the total amount of the sulfur-containing heterocyclic compound and the conjugated high molecular compound having a thiophenediyl group. Furthermore, when an electron-donating compound is contained in the solution, the amount of the electron-donating compound is preferably 1 to 100000 parts by weight, and more preferably 10 to 1000 parts by weight with respect to 100 parts by weight of the total amount of the sulfur-containing heterocyclic compound and the conjugated polymer compound having a thiophenediyl group.

The applied film is formed by applying the solution on one electrode.

Examples of the method of forming the film from the solution include a spin coating method, a casting method, a microgravure coating method, a gravure coating method, a bar coating method, a roll coating method, a wire bar coating method, a dip coating method, a spray coating method, a screen printing method, a flexographic printing method, an offset printing method, an ink jet printing method, a dispenser printing method, a nozzle coating method, and a capillary coating method. Among these, a spin coating method, a flexographic printing method, an ink jet printing method, and a dispenser printing method are preferable.

In the present invention, a temperature for drying an applied film to form an organic layer is 70°C or less, and preferably 0°C to 60°C. Drying may be carried out under an air atmosphere or under an inert atmosphere, or under a vacuum atmosphere. Preferably drying is carried out in an inert atmosphere. Furthermore, a drying time is preferably from 1 minute to 2 hours.

An organic photoelectric conversion element manufactured by the manufacturing method of the present invention has an organic layer. The organic layer includes a sulfur-containing heterocyclic compound, and a conjugated polymer compound having a thiophenediyl group. The weight of the sulfur-containing heterocyclic compound in the organic layer is preferably 0.1 to 10000 parts by weight, and more preferably 1 to 1000 parts by weight with respect to 100 parts by weight of the conjugated polymer compound having a thiophenediyl group.

The organic layer may further contain an electron-accepting compound. Examples of the electron-accepting compound include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, metal complexes of 8-hydroxyquinoline and derivatives thereof, polyquinoline and derivatives thereof, polyquinoxaline and derivatives thereof, polyfluorene and derivatives thereof, fullerenes such as C₆₀ fullerene and derivatives thereof, carbon nanotube, and phenanthroline derivatives such as 2,9-dimethyl-4,7-diphenyl-1,10-phenanthroline. Among them, fullerenes and derivatives thereof are preferable.

The fullerenes include C₆₀ fullerene, C₇₃ fullerene, and C₈₄ fullerene. The fullerene derivatives include C₆₀ fullerene derivatives, C₇₀ fullerene derivatives, and C₈₄ fullerene derivatives. The specific structures of the fullerene derivative include the following structures.

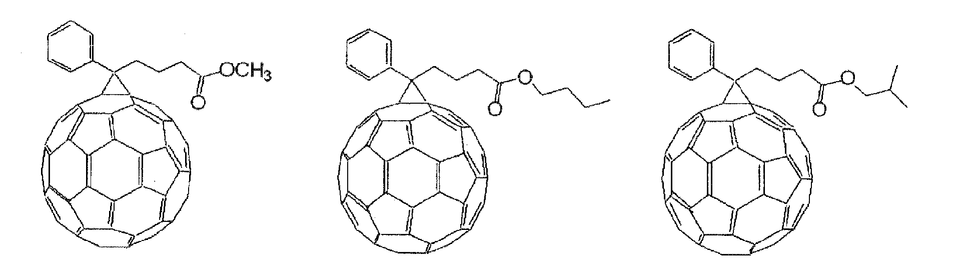

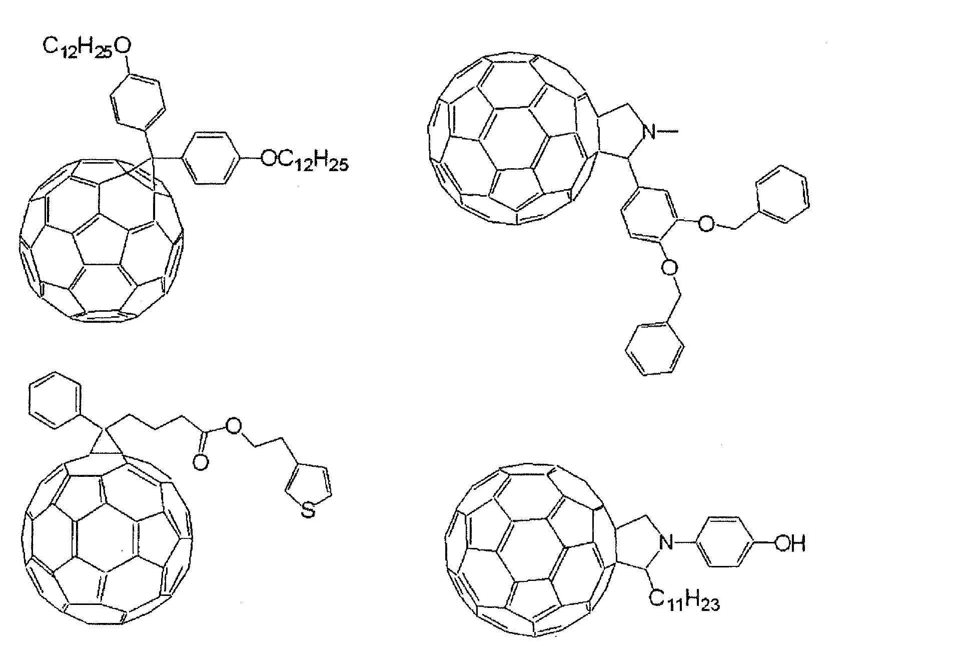

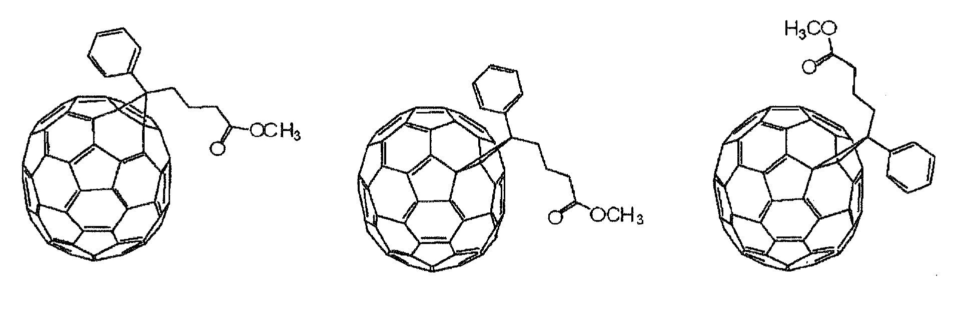

When the electron-accepting compound is contained, the amount of the electron-accepting compound in the organic layer is preferably 1 to 10000 parts by weight, and more preferably 10 to 2000 parts by weight with respect to 100 parts by weight of the total amount of the sulfur-containing heterocyclic compound and the conjugated polymer compound having a thiophenediyl group.

The organic layer may further contain an electron-donating compound. Examples of the electron-donating compound include pyrazoline derivatives, arylamine derivatives, stilbene derivatives, triphenyldiamine derivatives, oligothiophene and the derivatives thereof, polyvinylcarbazole and the derivatives thereof, polysilane and the derivatives thereof, polysiloxane derivatives having aromatic amine on the side chain or the main chain, polyaniline and the derivatives thereof, polythiophene and the derivatives thereof, polypyrrole and the derivatives thereof, polyphenylene vinylene and the derivatives thereof, and polythienylene vinylene and the derivatives thereof.

When the electron-donating compound is contained, the amount of the electron-donating compound in the organic layer is preferably 1 to 100000 parts by weight, and more preferably 10 to 1000 parts by weight with respect to 100 parts by weight of the total amount of the sulfur-containing heterocyclic compound and the conjugated polymer compound having a thiophenediyl group.

The organic layer may include components in addition to the sulfur-containing heterocyclic compound, the conjugated polymer compound having a thiophenediyl group, the electron-donating compound, and the electron-accepting compound within the scope in which the charge transport property and the charge injection property are not impaired.

The organic photoelectric conversion element manufactured by the manufacturing method of the present invention comprises a pair of electrodes at least one of which is transparent or translucent, and an organic layer which contains a sulfur-containing heterocyclic compound and a conjugated polymer compound having a thiophenediyl group as a repeating unit and which is disposed between the electrodes. A composition of the sulfur-containing heterocyclic compound and the conjugated polymer compound having a thiophenediyl group can be also used as an electron-accepting compound and as an electron-donating compound. Furthermore, when one of the sulfur-containing heterocyclic compound and the conjugated polymer compound having a thiophenediyl group is an electron-donating compound and the other is an electron-accepting compound, the composition may have functions of both of an electron donor and an electron acceptor. Among these embodiments, it is preferable that the composition is used as an electron-donating compound.

Next, an operation mechanism of the organic photoelectric conversion element will be described. The light energy incident from a transparent or translucent electrode is absorbed by an electron-accepting compound and/or an electron-donating compound to generate an exciton consisting of a bound electron-hole. When this exciton moves and reaches a hetero-junction interface where the electron-accepting compound and the electron-donating compound are present adjacent to each other, the electron and the hole are separated due to the difference of their HOMO energy and LUMO energy in the interface to generate charges (electron and hole) capable of moving separately. The generated charges can move to respective electrodes and can be taken outside as electric energy (current).

Specific examples of the organic photoelectric conversion element of the present invention include:
1. An organic photoelectric conversion element comprising a pair of electrodes, and a first organic layer containing a sulfur-containing heterocyclic compound and a conjugated polymer compound having a thiophenediyl group, and a second organic layer disposed adjacent to the first organic layer and containing an electron-donating compound, between the electrodes;
2. An organic photoelectric conversion element comprising a pair of electrodes, and a first organic layer containing an electron-accepting compound, and a second organic layer disposed adjacent to the first organic layer and containing a sulfur-containing heterocyclic compound and a conjugated polymer compound having a thiophenediyl group, between the electrodes;
3. An organic photoelectric conversion element comprising a pair of electrodes, and at least one organic layer containing a sulfur-containing heterocyclic compound, a conjugated polymer compound having a thiophenediyl group, and an electron-donating compound, between the electrodes;
4. An organic photoelectric conversion element comprising a pair of electrodes, and an organic layer containing a sulfur-containing heterocyclic compound, a conjugated polymer compound having a thiophenediyl group, and an electron-accepting compound, between the electrodes; and
5. An organic photoelectric conversion element comprising a pair of electrodes, and at least organic layer containing a sulfur-containing heterocyclic compound, a conjugated polymer compound having a thiophenediyl group, and an electron-accepting compound, between the electrodes, in which the electron-accepting compound is a fullerene derivative.

Furthermore, in the organic photoelectric conversion element described in the above 5, the weight of the fullerene derivative in the organic layer is preferably 10 to 1000 parts by weight and more preferably 50 to 500 parts by weight when the total weight of the weight of the sulfur-containing heterocyclic compound and the weight of the conjugated polymer compound having a thiophenediyl group is 100 parts by weight.

The organic photoelectric conversion element provided by the manufacturing method of the present invention is preferably the above 3, 4, or 5, and more preferably 5 in that it contains many hetero-junction interfaces. Furthermore, the organic photoelectric conversion element provided by the manufacturing method of the present invention may be provided with an additional layer between at least one of the electrodes and the organic layer of the element. Examples of the additional layer include a charge transport layer that transports a hole or an electron.

When a composition of a sulfur-containing heterocyclic compound and a conjugated polymer compound having a thiophenediyl group is used as an electron donor, in an electron acceptor suitably used for the organic photoelectric conversion element, the HOMO energy of the electron acceptor is higher than the HOMO energy of the conjugated high molecular compound having a thiophenediyl group and the HOMO energy of the sulfur-containing heterocyclic compound, and the LUMO energy of the electron acceptor is higher than the LUMO energy of the conjugated polymer compound having a thiophenediyl group and the LUMO energy of the sulfur-containing heterocyclic compound. Furthermore, when a composition of a sulfur-containing heterocyclic compound and a conjugated polymer compound having a thiophenediyl group is used as an electron acceptor, in an electron donor suitably used for the organic photoelectric conversion element, the HOMO energy of the electron donor is lower than the HOMO energy of the conjugated polymer compound having a thiophenediyl group and the HOMO energy of the sulfur-containing heterocyclic compound, and the LUMO energy of the electron donor is lower than the LUMO energy of the conjugated polymer compound having a thiophenediyl group and the LUMO energy of the sulfur-containing heterocyclic compound.

The organic photoelectric conversion element provided by the manufacturing method of the present invention is usually formed on a substrate. The substrate may be any material which does not change when the electrode is formed and the organic layer is formed. Examples of a material for the substrate include glass, plastic, polymer films, and silicon. When the substrate is an opaque substrate, an electrode opposite to the electrode provided at the substrate side (i.e., an electrode distal from the substrate) is preferably transparent or translucent.

As the transparent or translucent electrode material, a conductive metal oxide film, a translucent metal thin film, and the like are employed. Specific examples include indium oxide, zinc oxide, tin oxide, indium tin oxide (ITO) as a complex thereof, a film (NESA and the like) prepared by using a conductive material made of indium-zinc-oxide and the like, gold, platinum, silver, and copper. ITO, indium-zinc-oxide, and tin oxide are preferable. Examples of the method for producing an electrode include a vacuum vapor deposition method, a sputtering method, an ion plating method, and a plating method. Furthermore, an organic transparent conductive film of polyaniline and derivatives thereof, polythiophene and derivatives thereof, or the like may be used as the electrode material. Furthermore, a metal, a conductive high molecule, and the like, can be used as the electrode material. It is preferable that one of the pair of electrodes is made of a material with a small work function. Examples of the material include metals such as lithium, sodium, potassium, rubidium, cesium, magnesium, calcium, strontium, barium, aluminum, scandium, vanadium, zinc, yttrium, indium, cerium, samarium, europium, terbium, and ytterbium, and an alloy of two or more thereof, or an alloy of one or more thereof with one or more of gold, silver, platinum, copper, manganese, titanium, cobalt, nickel, tungsten, and tin, and graphite or a graphite interlayer compound. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy. Furthermore, it is preferable that one of the electrodes is a transparent electrode.

At the material used for a charge transport layer, that is, a hole transport layer and an electron transport layer as the additional layer, an electron-donating compound and an electron-accepting compound, mentioned later, respectively, can be used. The material used for a buffer layer as the additional layer can be halides of an alkali metal and an alkali earth metal, such as lithium fluoride, and oxides thereof and the like. Furthermore, fine particles of an inorganic semiconductor such as titanium oxide can be also used.

The method for manufacturing an organic photoelectric conversion element of the present invention comprises a step of applying a solution containing a conjugated polymer compound having a thiophenediyl group as a repeating unit and a sulfur-containing heterocyclic compound on one electrode to form an applied film. Herein, in applying of the solution on one electrode, the solution may be applied on the surface of the electrode, or an additional layer may be formed on the electrode and the solution may be applied on the surface of the additional layer, which is above the electrode.

As the organic layer in the organic photoelectric conversion element provided by the manufacturing method of the present invention, for example, an organic thin film containing a sulfur-containing heterocyclic compound and a conjugated polymer compound having a thiophenediyl group can be used.

The film thickness of the organic thin film is usually 1 nm to 100 µm, preferably 2 nm to 1000 nm, more preferably 5 nm to 500 nm, and further preferably 20 nm to 200 nm.

In order to enhance the hole transporting property of the organic thin film, a low molecular compound other than the sulfur-containing heterocyclic compound and/or a polymer other than the conjugated polymer compound having a thiophenediyl group as the electron-donating compound and/or the electron-accepting compound can be mixed and used in the organic thin film.

The organic photoelectric conversion element can generate photovoltaic power between electrodes by irradiation with light such as solar light through transparent or translucent electrodes, and is allowed to operate as an organic thin film solar battery. By accumulating a plurality of organic thin film solar batteries, the batteries can be used as an organic thin film solar battery module.

Photocurrent can flow by irradiation with light through transparent or translucent electrodes with voltage applied between the electrodes, and the organic photoelectric conversion element is allowed to operate as an organic light sensor. By accumulating a plurality of organic light sensors, the sensors can be used as an organic image sensor.

### EXAMPLES

Hereinafter, the present invention will be described in more detail by examples, but the present invention is not limited thereto.

### <Example 1>

Regioregular poly-3-hexylthiophene (manufactured by Sigma-Aldrich Corporation, Mn: ∼64000, hereinafter, referred to as "P3HT") was dissolved in chlorobenzene at the concentration of 1% by weight. Furthermore, 1 part by weight of C60PCBM (Phenyl C61-butyric acid methyl ester, manufactured by American Dye Source, Inc., trade name: ADS61BFB) with respect to 1 part by weight of P3HT was mixed as an electron acceptor in the solution. After that, 5 parts by weight of 2, 2' -bithiophene with respect to 1 part by weight of P3HT was mixed in the solution. Next, the solution was filtrated through a Teflon (registered trademark) filter having a pore size of 0.2 µm to produce an applying solution. The obtained applying solution was applied on a glass substrate by spin coating. The applying operation was carried out at 23°C. After that, drying was carried out in a vacuum atmosphere at 23°C for 60 minutes to obtain an organic thin film having a film thickness of about 100 nm. The absorption spectrum of the organic thin film was measured by using a spectrophotometer (manufactured by JASCO Corporation, trade name: V-670). The absorbance at 600 nm is shown in Table 1.

### <Example 2>

An organic thin film was produced in the same manner as in Example 1 except that 1 part by weight of 2,2'-bithiophene with respect to 1 part by weight of P3HT was mixed in the solution, and the absorption spectrum of the organic thin film was measured. The absorbance at 600 nm is shown in Table 1.

### <Example 3>

An organic thin film was produced in the same manner as in Example 1 except that 0.5 parts by weight of 2,2' -bithiophene with respect to 1 part by weight of P3HT was mixed in the solution, and the absorption spectrum of the organic thin film was measured. The absorbance at 600 nm is shown in Table 1.

### <Example 4>

An organic thin film was produced in the same manner as in Example 1 except that 1 part by weight of 2,2':5',2"-terthiophene instead of 2, 2' -bithiophene with respect to 1 part by weight of P3HT was mixed in the solution, and the absorption spectrum of the organic thin film was measured. The absorbance at 600 nm is shown in Table 1.

### <Example 5>

An organic thin film was produced in the same manner as in Example 1 except that 1 part by weight of Compound (A) instead of 2,2'-bithiophene with respect to 1 part by weight of P3HT was mixed in the solution, and the absorption spectrum of the organic thin film was measured. The absorbance at 600 nm is shown in Table 1.

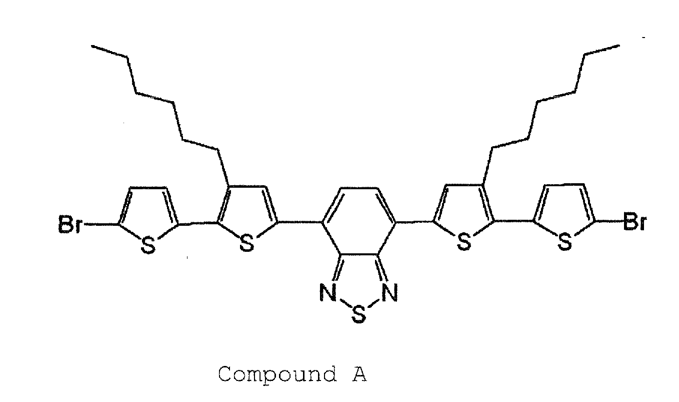

### <Example 6>

An organic thin film was produced in the same manner as in Example 1 except that 1 part by weight of 3, 3' -bithiophene instead of 2,2'-bithiophene with respect to 1 part by weight of P3HT was mixed in the solution, and the absorption spectrum of the organic thin film was measured. The absorbance at 600 nm is shown in Table 1.

### <Comparative Example 1>

An organic thin film was produced in the same manner as in Example 1 except that an applying solution was produced without adding 2,2'-bithiophene, and the absorption spectrum of the organic thin film was measured. The absorbance at 600 nm is shown in Table

**[Table 1]**

| | Sulfur-containing heterocyclic compound | Addition amount (with respect to P3HT, % by weight | Absorbance at 600 nm |
|---|---|---|---|
| Example 1 | 2,2'-bithiophene | 500 | 0.28 |
| Example 2 | 2,2'-bithiophene | 100 | 0.25 |
| Example 3 | 2,2'-bithiophene | 50 | 0.22 |
| Example 4 | 2,2':5',2" -terthiophene | 100 | 0.22 |
| Example 5 | Compound A | 100 | 0.18 |
| Example 6 | 3,3'-bithiophene | 100 | 0.22 |
| Comparative Example 1 | None | | 0.11 |

### <Example 7>

### (Production and Evaluation of Organic Thin Film Solar Battery)

P3HT was dissolved in chlorobenzene at the concentration of 1% by weight. After that, 5 parts by weight of 2,2'-bithiophene with respect to 1 part by weight of P3HT was mixed in the solution. Furthermore, 1 part by weight of C60PCBM (Phenyl C61-butyric acid methyl ester, manufactured by American Dye Source, Inc., trade name: ADS61BFB) with respect to 1 part by weight of P3HT was mixed as an electron acceptor in the solution. Next, the solution was filtrated through a Teflon (registered trademark) filter having a pore size of 0.2 µm to produce an applying solution.

A glass substrate provided with a 150 nm-thick ITO film formed by a sputtering method was surface-treated by ozone UV treatment. Next, the applying solution was applied by spin coating to form an applied film. After that, drying was carried out in a vacuum atmosphere for 60 minutes to obtain an organic layer being an active layer (film thickness: about 100 nm) of an organic thin film solar battery. The applying operation and the drying were carried out at 23°C. Heating operation for increasing the temperature to higher than 23°C was not carried out. After that, by using a vacuum vapor deposition machine, lithium fluoride was vapor deposited at a film thickness of 4 nm, and then Al was vapor deposited at a film thickness of 100 nm. The degree of vacuum in vapor deposition was 1 to 9 × 10⁻³ Pa in all cases. Furthermore, the shape of the obtained organic thin film solar battery was 2 mm × 2 mm square. The obtained organic thin film solar battery was irradiated with a predetermined light by using Solar Simulator (manufactured by Bunkoukeiki Co., Ltd., trade name: OTENTO-SUNII: AM1.5G filter, irradiance: 100 mW/cm²), and the current and voltage generated were measured so as to obtain the photoelectric conversion efficiency. The results are shown in Table 2.

### <Example 8>

An organic thin film solar battery was produced in the same manner as in Example 7 except that 1 part by weight of 2, 2' -bithiophene with respect to 1 part by weight of P3HT was mixed in the solution, and the photoelectric conversion efficiency was obtained. The results are shown in Table 2.

### <Example 9>

An organic thin film solar battery was produced in the same manner as in Example 7 except that 0.5 parts by weight of 2, 2' -bithiophene with respect to 1 part by weight of P3HT was mixed in the solution, and the photoelectric conversion efficiency was obtained. The results are shown in Table 2.

### <Comparative Example 2>

An organic thin film solar battery was produced in the same manner as in Example 7 except that an applying solution was produced without adding 2,2'-bithiophene, and the photoelectric conversion efficiency was obtained. The results are shown in Table 2.

**[Table 2]**

| | Sulfur-containing heterocyclic compound | Addition amount (with respect to P3HT, % by weight | Conversion efficiency (%) |
|---|---|---|---|
| Example 7 | 2,2'-bithiophene | 500 | 2.3 |
| Example 8 | 2,2'-bithiophene | 100 | 2.6 |
| Example 9 | 2,2'-bithiophene | 50 | 2.3 |
| Comparative Example 2 | None | | 1.1 |

### INDUSTRIAL APPLICABILITY

A method for manufacturing an organic photoelectric conversion element of the present invention can form an organic layer at a low temperature and can provide an organic photoelectric conversion element having a high absorbance at 600 nm, and it is therefore extremely useful.

## Claims

1. A method for manufacturing an organic photoelectric conversion element having a pair of electrodes at least one of which is transparent or translucent, and an organic layer between the electrodes, the method comprising:
a step of applying a solution that contains a conjugated polymer compound having a thiophenediyl group as a repeating unit and a sulfur-containing heterocyclic compound on one of the electrodes to form an applied film, and
a step of drying the applied film at a temperature of 70°C or less to form the organic layer.

2. The method for manufacturing an organic photoelectric conversion element according to claim 1, comprising a step of drying the applied film at a temperature of 70°C or less in a vacuum atmosphere to form the organic layer.

3. The method for manufacturing an organic photoelectric conversion element according to claim 1, wherein the sulfur-containing heterocyclic compound is a compound represented by formula (1): 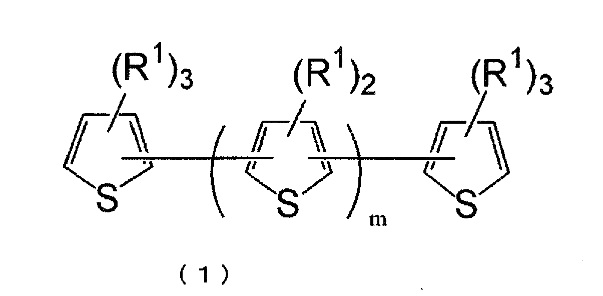 wherein a plurality of R¹s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group; a hydrogen atom or atoms contained in these groups may be substituted with a fluorine atom or atoms; and m denotes an integer from 0 to 10.

4. The method for manufacturing an organic photoelectric conversion element according to claim 1, wherein the sulfur-containing heterocyclic compound is 2,2'-bithiophene, and the conjugated polymer compound is regioregular poly-3-substituted thiophene.

5. The method for manufacturing an organic photoelectric conversion element according to claim 1, wherein the conjugated polymer compound is a polymer compound which has a polystyrene-equivalent weight average molecular weight of 5 × 10² to 1 × 10⁷ and which has a repeating unit represented by formula (6) or the repeating unit represented by formula (6) and a repeating unit represented by formula (7): 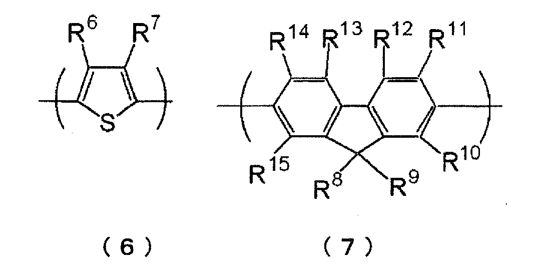 wherein R⁶, R⁷, R⁸, R⁹, R¹⁰, R¹¹, R¹², R¹³, R¹⁴, and R¹⁵ independently denote a hydrogen atom, an alkyl group, an alkoxy group, an alkylthio group, an aryl group, an aryloxy group, an arylthio group, an arylalkyl group, an arylalkoxy group, or an arylalkylthio group.

6. The method for manufacturing an organic photoelectric conversion element according to claim 1, wherein the sulfur-containing heterocyclic compound is a compound represented by formula (2): 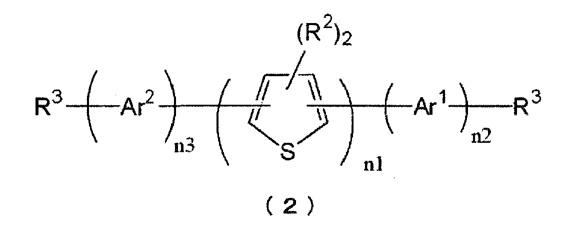 wherein a plurality of R²s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group; a hydrogen atom or atoms contained in these groups may be substituted with a fluorine atom or atoms; a plurality of R³s may be the same or different, and denote a hydrogen atom, a halogen atom, an alkyl group, an alkoxy group, or an alkylthio group; a hydrogen atom or atoms contained in these groups may be substituted with a fluorine atom or atoms; Ar¹ and Ar² are the same or different and denote an arylene group or a divalent nitrogen-containing aromatic heterocyclic group; n1 denotes an integer from 2 to 10; n2 denotes an integer from 1 to 3, and n3 denotes an integer from 1 to 3; when a plurality of Ar¹s are present, they may be the same or different; and when a plurality of Ar²s are present, they may be the same or different.

7. The method for manufacturing an organic photoelectric conversion element according to claim 1, wherein the sulfur-containing heterocyclic compound is a compound represented by formula (3): 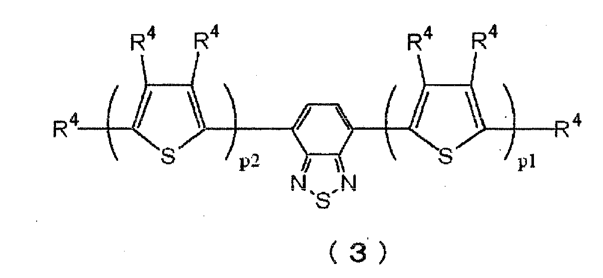 wherein a plurality of R⁴s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group; a hydrogen atom or atoms contained in these groups may be substituted with a fluorine atom or atoms; p1 denotes an integer from 0 to 5, and p2 denotes an integer from 0 to 5.

8. The method for manufacturing an organic photoelectric conversion element according to claim 1, wherein the sulfur-containing heterocyclic compound is represented by formula (4) : 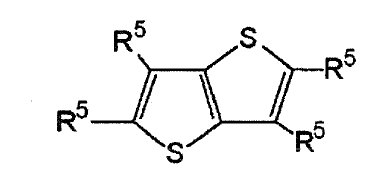 wherein a plurality of R⁵s may be the same or different, and denote a hydrogen atom, a halogen atom, an alkyl group, an alkoxy group, an alkylthio group, an aryl group, an aryloxy group, an arylthio group, an arylalkyl group, an arylalkoxy group, or an arylalkylthio group; and a hydrogen atom or atoms contained in these groups may be substituted with a fluorine atom or atoms.

9. The method for manufacturing an organic photoelectric conversion element according to claim 1, wherein the proportion of the sulfur-containing heterocyclic compound in the organic layer is 0.1 to 10000 parts by weight with respect to 100 parts by weight of the conjugated high molecular compound.

10. The method for manufacturing an organic photoelectric conversion element according to claim 1, wherein an electron-accepting compound is further contained in the organic layer.

11. The method for manufacturing an organic photoelectric conversion element according to claim 10, wherein the electron-accepting compound is fullerene or a fullerene derivative.

12. The method for manufacturing an organic photoelectric conversion element according to claim 1, wherein an electron-donating compound is further contained in the organic layer.
